# EUROPEAN PATENT APPLICATION

(11) **EP 4 822 353 A2**
(43) Date of publication of application: **14.10.2026**
(21) Application number: 26193634.8
(22) Date of filing: 25.05.2022
(51) Int. Cl.: G01N 33/50

(54) **NOVEL NEURONAL CELL LINE FOR MEASURING TITER OF NEUROTOXIN**

(30) Priority: 25.05.2021 KR 20210066781
(62) Document number(s) of the earlier application(s) in accordance with Art. 76 EPC: 22811635.6 / 4 349 971
(71) Applicant: Hugel Inc., Gangwon-do 24206 (KR)
(72) Inventor: Jeon, Heesoo, 08737 Seoul (KR); Choi, Yusun, 24399 Chuncheon-si Gangwon-do (KR); Oum, Ji-Hyun, 13550 Seongnam-si Gyeonggi-do (KR); Lee, Changki, 24214 Chunchseon-si Gangwon-do (KR); Oh, Donghoon, 24419 Chuncheon-si Gangwon-do (KR); Lee, Changjin, 04194 Soul (KR); Kim, Sena, 24448 Chuncheon-si Gangwon-do (KR)
(74) Representative: Latscha Schöllhorn Partner AG

(57) **Abstract**

As botulinum toxin has recently been attempted as a therapeutic agent for various indications, the demand for botulinum toxin in the medical and cosmetic fields has rapidly increased. However, there is no stable and highly reproducible cell-based assay method for measuring the potency of botulinum toxin. Since botulinum toxin is a very potent neurotoxin protein, the development of highly specific and sensitive cells is particularly required for accurate cell-based measurement of the potency of botulinum toxin. The present invention is directed to a neuronal cell line with altered gene expression. The transformed cell line according to the present invention has significantly increased sensitivity to botulinum toxin, and thus is expected to be very effectively used for cell-based determination of botulinum toxin activity or cell-based detection of botulinum toxin.

## Description

### Technical Field

The present invention relates to a novel neuronal cell line for measuring the potency of neurotoxin.

### Background Art

Botulinum toxin is a neurotoxic protein produced by the bacterium *Clostridium botulinum*. Functionally, botulinum toxin inhibits the release of the neurotransmitter acetylcholine at the presynapse of the neuromuscular junction, causing flaccid paralysis of muscles. Previously, botulinum toxin was known as a cause of botulism. However, attempts have recently been made to use the muscle-paralyzing mechanism of botulinum toxin to treat various indications, such as optic diseases, various autonomic nervous system disorders, including pain and sweat gland disorders, migraine headache pain, postoperative pain and visceral pain, skin diseases such as psoriasis, neurogenic inflammation, and mental illness disorders.

However, botulinum toxin is the most lethal substance among biotoxins known to mankind, and the lethal dose (LD₅₀) of botulinum toxin in humans is 1.3 to 2.1 ng/kg when injected intravenously or intramuscularly, and 10 to 13 ng/kg when inhaled. As such, botulinum toxin has a great therapeutic effect on various diseases. However, botulinum toxin is highly toxic and is fatal even in very small amounts, and thus when it is to be used in living organisms, the fine control of the concentration thereof is essential. To this end, Allergan, the manufacturer of Botox^{®}, successfully established the human neuroblastoma cell line SiMa as a cell line for measuring the potency of botulinum toxin (PLoS One. 2012;7(11):e49516). However, the SiMa cell line grows very slowly, generally requiring a population doubling time (PDT) of 70 hours or more. Moreover, the generation of iPS cells is also very time-consuming, and the storage thereof is even more difficult. In addition, Dr. David Beebe's group at the University of Wisconsin noted that there is a problem with the suitability of SiMa as a cell line for measuring the potency of botulinum toxin because the SiMa cell line does not exhibit motor neuron-like characteristics (J Biomol Screen. 2016 Jan;21(1):65-73). Therefore, there has been a need to develop a neuronal cell line for measuring the potency of botulinum toxin, which is highly sensitive to botulinum toxin, has a short PDT, and may be easily stored.

The present invention has been made in order to solve the above-described problems and is directed to a novel neuronal cell line having significantly improved sensitivity to botulinum toxin. It is expected that the novel cell line of the present invention will be actively used for botulinum toxin research.

### DISCLOSURE

### Technical Problem

An object of the present invention is to provide a novel neuronal cell line obtained by transformation so that the cell line is sensitive to botulinum toxin.

Another object of the present invention is to provide a cell-based method of determining the activity of botulinum toxin using the above cell line.

Still another object of the present invention is to provide a cell-based method of detecting botulinum toxin using the above cell line.

However, objects to be achieved by the present invention are not limited to the objects mentioned above, and other objects not mentioned above may be clearly understood by those skilled in the art from the following description.

### Technical Solution

Hereinafter, various embodiments described herein will be described with reference to figures. In the following description, numerous specific details are set forth, such as specific configurations, compositions, and processes, etc., in order to provide a thorough understanding of the present invention. However, certain embodiments may be practiced without one or more of these specific details, or in combination with other known methods and configurations. In other instances, known processes and preparation techniques have not been described in particular detail in order to not unnecessarily obscure the present invention. Reference throughout this specification to "one embodiment" or "an embodiment" means that a particular feature, configuration, composition, or characteristic described in connection with the embodiment is included in at least one embodiment of the present invention. Thus, the appearances of the phrase "in one embodiment" or "an embodiment" in various places throughout this specification are not necessarily referring to the same embodiment of the present invention. Additionally, the particular features, configurations, compositions, or characteristics may be combined in any suitable manner in one or more embodiments.

Unless otherwise stated in the specification, all the scientific and technical terms used in the specification have the same meanings as commonly understood by those skilled in the art to which the present invention pertains.

As botulinum toxin has recently been attempted as a therapeutic agent for various indications, the demand for botulinum toxin in the medical and cosmetic fields has rapidly increased. However, there is no stable and highly reproducible cell-based assay method for measuring the potency of botulinum toxin. Since botulinum toxin is a very potent neurotoxin protein, the development of highly specific and sensitive cells is particularly required for accurate cell-based measurement of the potency of botulinum toxin.

In one embodiment of the present invention, "botulinum toxin" is a neurotoxic protein produced by the bacterium *Clostridium botulinum*. The genus *Clostridium* has more than 127 species, grouped according to their morphology and functions. The anaerobic, gram-positive bacterium *Clostridium botulinum* produces a potent polypeptide neurotoxin, botulinum toxin, which causes a neuroparalytic illness in humans and animals referred to as botulism. The spores of *Clostridium botulinum* are found in soil and can grow in improperly sterilized and sealed food containers of home based canneries, which are the cause of many of the cases of botulism. The symptoms of botulism typically appear 18 to 36 hours after eating the foodstuffs infected with a *Clostridium botulinum* culture or spores. Botulinum toxin can apparently pass unattenuated through the lining of the gut and shows a high affinity for cholinergic motor neurons. Symptoms of botulinum toxin intoxication can progress from difficulty in walking, swallowing, and speaking to paralysis of the respiratory muscles and death. Botulinum toxins have been used in clinical settings for the treatment of neuromuscular disorders characterized by hyperactive skeletal muscles (i.e. motor disorders). In 1989, a botulinum toxin type A complex was approved by the U.S. Food and Drug Administration for the treatment of essential blepharospasm, strabismus and hemifacial spasm. Subsequently, botulinum toxin type A was also approved by the FDA for the treatment of cervical dystonia and for the treatment of glabellar lines, and botulinum toxin type B was also approved for the treatment of cervical dystonia. Non-type A botulinum toxin serotypes apparently have a lower potency and/or a shorter duration of activity as compared to botulinum toxin type A. Clinical effects of peripheral intramuscular botulinum toxin type A are usually seen within one week of injection. The typical duration of symptomatic relief from a single intramuscular injection of botulinum toxin type A averages about 3 months, although significantly longer periods of therapeutic activity have been reported. Although all the botulinum toxin serotypes apparently inhibit release of the neurotransmitter acetylcholine at the neuromuscular junction, they do so by affecting different neurosecretory proteins and cleaving these proteins at different sites. For example, botulinum types A and E both cleave the 25-kDa synaptosomal associated protein (SNAP-25), but they target different amino acid sequences within this protein. Botulinum toxin types B, D, F and G act on vesicle-associated membrane protein (VAMP, also called synaptobrevin), with each serotype cleaving the protein at a different site. Finally, botulinum toxin type C1 appears to cleave both syntaxin and SNAP-25. These differences in mechanism of action may affect the relative potency and/or duration of action of the various botulinum toxin serotypes. Particularly, a substrate for a botulinum toxin can be found in a variety of different cell types. Moreover, *in vitro* studies have indicated that botulinum toxin inhibits potassium cation-induced release of both acetylcholine and norepinephrine from primary cell cultures of brainstem tissue. In addition, it has been reported that botulinum toxin inhibits the evoked release of both glycine and glutamate in primary cultures of spinal cord neurons and that in brain synaptosome preparations, botulinum toxin inhibits the release of each of the neurotransmitters acetylcholine, dopamine, norepinephrine, CGRP, substance P, and glutamate. Thus, when adequate concentrations are used, the stimulus-evoked release of most neurotransmitters can be blocked by botulinum toxin.

In one embodiment of the present invention, "activity of toxin" means the potency of botulinum toxin. One unit (U) of botulinum toxin is defined as the amount of botulinum toxin that kills 50% of a group of mice, each weighing 18 to 20 g, when measured by mouse LD50 bioassay (mLD50), a standard assay method. Botulinum toxin, particularly botulinum toxin serotype A, is the most lethal natural biological agent known to man, and is 1.8 billion times more lethal than diphtheria toxin, 600 million times more lethal than sodium cyanide, 30 million times more lethal than cobra toxin, and 12 million times more lethal than cholera toxin. Thus, a difference in potency of about 20% in botulinum toxin results in a significant difference in effect, such as 360 million times diphtheria toxin, or 2.4 million times cholera toxin.

Botulinum toxin formulations for medical or cosmetic purposes are generally distributed as lyophilized formulations or liquid formulations, and have a problem in that because botulinum toxin itself is a protein, the activity thereof becomes very unstable by temperature, pH, light, physical impact, or gas (air, nitrogen, oxygen, etc.). When the potency of botulinum toxin is reduced as described above, it hardly exhibits its expected effect, and hence it is necessarily required to accurately predict the potency of botulinum toxin in a preparation step or a use step.

The present inventors have developed a novel neuronal cell line sensitive to botulinum toxin by performing transformation so that any one or more genes selected from the group consisting of Stxbp2 (syntaxin-binding protein 2), Stxbp6 (syntaxin-binding protein 6), DNAJC5 (DnaJ homolog subfamily C member 5), Stx5a (syntaxin 5A), and KCNA4 (potassium voltage-gated channel subfamily A member 4) are overexpressed compared to those in the parent cell line, or by performing transformation so that the expression of any one or more genes selected from the group consisting of NAPA (NSF attachment protein alpha)-a, NAPA-b, NAPA-c, NAPB (NSF attachment protein beta)-a, NAPB-b, and NAPB-c is expressed compared to that in the parent cell line.

The "transformation" refers to means inserting a specific DNA fragment into the genome of a living organism so that a new genetic trait is expressed. The term "transformation" is considered to have the same meaning as the term "transduction" or "transfection" in the sense of introducing a new trait. Transformation in the present invention includes manipulating neurons as parent cells so as to overexpress or silence a specific trait so that the sensitivity of the cells to botulinum toxin is increased.

The cell line of the present invention may be used to determine the activity of botulinum toxin or to detect botulinum toxin.

The cell line of the present invention maintains its sensitivity to botulinum toxin even if the passage thereof continues. Thus, the cell line may be used very suitably in a cell-based assay platform.

The cell line for determining the activity of botulinum toxin according to the present invention means homogeneous single cells isolated from a parental neuronal cell line corresponding to a population including various types of cells, and refers to cells having common genetic features, for example, high or low expression levels of a specific gene, or the like.

The parental neuronal cell line of the present invention may include any immortalized cell line derived from nerve, and may be preferably Neuro-2a cells or SiMa cells, more preferably Neuro-2a (ATCC^{®} CCL-131^{™}) or SiMa cells (DSMZ^{®} ACC 164^{TM}), without being limited thereto.

The cell line for determining the activity of botulinum toxin according to the present invention may be used to detect botulinum toxin or determine the activity thereof. Since the cell line is sensitive to botulinum toxin, it can detect the presence or absence of botulinum toxin in a sample of interest, and can also measure the degree of toxicity of botulinum toxin depending on the concentration of botulinum toxin.

The botulinum toxin of the present invention is a neurotoxic protein produced by the bacterium *Clostridium botulinum*, and may be classified into a total of seven serotypes: A, B, C (C1, C2), D, E, F and G. The botulinum toxin affects different neurosecretory proteins depending on the serotypes and cleaves these proteins at different sites. Specifically, both botulinum toxin serotypes A and E can cleave SNAP25 (synaptosomal nerve-associated protein 25), botulinum toxin serotypes B, D, F and G can cleave VAMP (vesicle-associated membrane protein), and botulinum toxin serotype C1 can cleave both syntaxin and SNAP25, thereby inducing neurotoxicity. Preferably, the botulinum toxin may be botulinum toxin serotype A or botulinum toxin serotype E, without being limited thereto.

The botulinum toxin serotype A of the present invention is purified and widely used for treatment of dystonia, aesthetic applications, and the like. Thus, when the cell line for determining the activity of botulinum toxin according to the present invention is used to measure the potency of botulinum toxin, there is an advantage in that the concentration at which side effects can occur can be determined, thereby solving the problems which can occur when the botulinum toxin is used for the above-described applications.

Another embodiment of the present invention provides a method for producing a neuronal cell line sensitive to botulinum toxin.

The method of the present invention comprises steps of: preparing a neuronal cell line as a parent cell line; and transforming the neuronal cell line so that any one or more genes selected from the group consisting of Stxbp2 (syntaxin-binding protein 2), Stxbp6 (syntaxin-binding protein 6), DNAJC5 (DnaJ homolog subfamily C member 5), Stx5a (syntaxin 5A), and KCNA4 (potassium voltage-gated channel subfamily A member 4) are overexpressed or any one or more genes selected from the group consisting of NAPA (NSF attachment protein alpha)-a, NAPA-b, NAPA-c, NAPB (NSF attachment protein beta)-a, NAPB-b, and NAPB-c are silenced.

In the method for producing a neuronal cell line sensitive to botulinum toxin according to the present invention, the neuronal cell line as a parent cell line has the same meaning as the parental neural cell line described above with respect to the novel neural cell line sensitive to botulinum toxin, and thus the description of the contents related to the parental neuronal cell line, the botulinum toxin, etc. will be omitted to avoid excessive complexity of the specification due to repeated description thereof.

Still another embodiment of the present invention provides a cell-based method for determining the activity of botulinum toxin.

The method of the present invention comprises steps of: culturing the cell line according to the present invention; treating the cultured cell line with the botulinum toxin; and measuring the sensitivity of the botulinum toxin-treated cell line to the botulinum toxin.

The cell-based method for determining the activity of botulinum toxin according to the present invention may be achieved by treating the cell line for determining the activity of botulinum toxin with the botulinum toxin, and measuring the sensitivity of the cell line to the botulinum toxin. Thus, the description of the contents related to the cell line for determining the activity of botulinum toxin, the botulinum toxin, the neurosecretory proteins cleaved by the botulinum toxin, the parental neuronal cell line, and the like, will be omitted in order to avoid excessive complexity of the specification due to repeated description thereof.

The step of measuring the sensitivity of the cell line to the botulinum toxin in the present invention may comprise measuring the cleavage of endogenous neurosecretory protein caused by the botulinum toxin. Specifically, in the case of botulinum toxin serotype A and serotype E, the cleavage of SNAP25 may be measured, and in the case of botulinum toxin serotypes B, D, F and G, the cleavage of VAMP may be measured, and in the case of botulinum toxin serotype C1, the cleavage of syntaxin and/or SNAP25 may be measured.

The measurement of the cleavage in the present invention may be achieved through a method of detecting a protein using an antibody specific for the cleaved peptide of the endogenous neurosecretory protein, or the like.

The term "antibody" means a protein molecule that can recognize the whole or cleaved peptide of the neurosecretory protein as an antigen and can bind specifically thereto. Examples of the antibody include polyclonal antibodies, monoclonal antibodies, and recombinant antibodies.

The method of detecting the protein, which is used in the present invention, may be any conventional method for detecting protein, and examples thereof include, but are not limited to, Western blotting assay, ELISA (enzyme-linked immunosorbent assay), RIA (radioimmunoassay), radioimmunodiffusion, Ouchterlony immunodiffusion, rocket immunoelectrophoresis, immunohistostaining, immunoprecipitation assay, complement fixation assay, fluorescence activated cell sorter (FACS) assay, protein chip assay, and the like.

Yet another embodiment of the present invention provides a cell-based method for detecting botulinum toxin.

The method of the present invention comprises steps of: culturing the cell line according to the present invention; treating the cultured cell line with a sample of interest; and measuring the sensitivity of the sample-treated cell line to the botulinum toxin.

The cell-based method for detecting botulinum toxin according to the present invention may be achieved by treating the cell line with the sample of interest instead of the botulinum toxin used in the cell-based method for determining the activity of botulinum toxin, and measuring the sensitivity of the cell line to the botulinum toxin. Thus, the description of the contents related to the cell line for determining the activity of botulinum toxin, the botulinum toxin, the neurosecretory proteins cleaved by the botulinum toxin, the parental neuronal cell line, the protein detection method, the antibody, and the like will be omitted in order to avoid excessive complexity of the specification due to repeated description thereof.

The sample of interest which is used in the present invention is a sample expected to contain botulinum toxin, and examples thereof include biological samples, including cell culture supernatants, blood, saliva, sputum, cerebrospinal fluids, secretions, lymphatic fluids, dialysis fluids, body fluids, urine and the like, and chemical samples containing compounds.

The transformed neuronal cell line provided by the present invention is defined as having sensitivity to botulinum toxin serotype A, which is different from the sensitivity of the parental neural cell line (parent cell line). The neuronal cell line of the present invention is one obtained by performing transformation so that any one or more genes selected from the group consisting of Stxbp2, Stxbp6, DNAJC5, Stx5a, and KCNA4 are overexpressed compared to those in the parent cell line, or by performing transformation so that any one or more genes selected from the group consisting of NAPA-a, NAPA-b, NAPA-c, NAPB-a, NAPB-b, and NAPB-c are silenced compared to those in the parent cell line. The neuronal cell line of the present invention has increased sensitivity to botulinum toxin serotype A compared to the parental cell line, and preferably has at least 15% higher sensitivity to botulinum toxin serotype A than the parental cell line. This feature can be measured by treating the cells with botulinum toxin and measuring the percentage of botulinum toxin-induced cleavage of SNAP25 protein in the cells.

Still yet another embodiment of the present invention provides a cell composition for measuring the activity of botulinum toxin, the cell composition comprising any one or more cells described above.

Hereinafter, each step of the present invention will be described in detail.

### Advantageous Effects

The present invention is directed to a neuronal cell line with altered gene expression. The transformed cell line according to the present invention has significantly increased sensitivity to botulinum toxin by overexpressing or silencing a specific gene.

### Brief Description of Drawings

FIG. 1 shows the results of cleaving SNAP25 protein by treatment with botulinum neurotoxin type A (BoNT/A) in Neuro-2a cells transfected with an expression vector or knock-down vector, according to one example of the present invention.
FIGS. 2a to 2c show the results of cleaving SNAP25 protein by treatment with botulinum neurotoxin type A (BoNT/A) in Neuro-2a cells transfected with SV2C, Syt1, SNAP25, Stxbp2, Stxbp6, DNAJC5, Stx5a, or KCNA4 gene, according to one example of the present invention.
FIGS. 3a to 3d show the results of cleaving SNAP25 protein by treatment with botulinum neurotoxin type A (BoNT/A) in Neuro-2a cells single-transfected with NAPA-a, NAPB-a, NAPA-b, NAPB-b, NAPA-c, or NAPB-c, or co-transfected with NAPA-a + NAPB-a, NAPA-b + NAPB-b, NAPA-c + NAPB-c, NAPA-b + NAPA-c, or NAPB-b + NAPB-c, according to one example of the present invention.
FIG. 4 shows the results of cleaving SNAP25 protein by treatment with botulinum neurotoxin type A (BoNT/A) in SiMa cells transfected with an expression vector or knock-down vector, according to one example of the present invention.
FIGS. 5a and 5b show the results of cleaving SNAP25 protein by treatment with botulinum neurotoxin type A (BoNT/A) in Neuro-2a cells transfected with Stxbp2, Stxbp6, DNAJC5, Stx5a, or KCNA4 gene, according to one example of the present invention.
FIGS. 6a to 6c show the results of cleaving SNAP25 protein by treatment with botulinum neurotoxin type A (BoNT/A) in Neuro-2a cells single-transfected with NAPA-b, NAPB-b, NAPA-c, or NAPB-c, or co-transfected with NAPA-b + NAPB-b, NAPA-c + NAPB-c, NAPA-b + NAPA-c, or NAPB-b + NAPB-c, according to one example of the present invention.

### Mode for Invention

Hereinafter, the present invention will be described in more detail with reference to examples. These examples are only for illustrating the present invention in more detail, and it will be apparent to those skilled in the art that the scope of the present invention according to the subject matter of the present invention is not limited by these examples.

### Experimental Examples

### 1. Selection of Target Genes Involved in Sensitivity to Botulinum Toxin

Gene expression patterns in Neuro-2a cells (ATCC^{®} CCL-131^{™}) and N2-42F cells (KCTC 13712BP) were compared using Affymetrix GeneChip^{®} Mouse Gene 2.0 ST Oligonucleotide Array and Ingenuty^{®} Pathway Analysis (IPA^{®}, QIAGEN Inc.). N2-42F cells are cells selected from Neuro-2a cells through a clonal selection process and are cells developed to be remarkably sensitive to botulinum neurotoxin type A (BoNT/A). The present inventors selected genes whose expression levels were at least 1.4 times higher or lower than those in Neuro-2a cells, and then selected 10 genes, which were associated with SNAP25 (synaptosome associated protein 25) and determined to be involved in improving sensitivity to botulinum neurotoxin type A (BoNT)/A). The selected genes are shown in Table 1 below.

**[Table 1]**

| **Gene** | **Expression** | | **Description** | **Species** | **Accession number** |
|---|---|---|---|---|---|
| | **Up/ Down** | **Fold change (N2-42F/ Neuro-2a)** | | | |
| SV2C | Up | 1.840 | BoNT/A target | Mus musculus | NM_029210 |
| Syt1 | Up | 1.730 | BoNT/B target | Mus musculus | NM_001252341 |
| SNAP25 | Up | 1.413 | BoNT/A target | Mus musculus | NM_001291056 |
| Stxbp2 | Up | 1.504 | Regulator of SNARE complex | Mus musculus | NM_011503 |
| Stxbp6 | Up | 1.457 | Regulator of SNARE complex | Mus musculus | NM_144552 |
| DNAJC5 | Up | 1.569 | Synaptogenesis signaling pathway | Mus musculus | NM_001271584.1 |
| Stx5a | Up | 1.447 | Synaptic vesicle fusion | Mus musculus | NM_001167799 |
| KCNA4 | Up | 3.324 | Neurotransmitter release | Mus musculus | NM_021275 |
| NAPA | Down | 0.635 | Regulator of SNARE complex | Mus musculus | NM_025898 |
| NAPB | Down | 0.621 | Regulator of SNARE complex | Mus musculus | NM_019632 |

### 2. Gene Cloning

Cells were single-transfected or co-transfected with the genes shown in Table 1. For co-transfection, two of types a, b, and c of each of NAPA and NAPB were selected. Eight genes, excluding Syt1 and DNAJC5, were cloned using the plasmid vector purchased from Genecopoiea Inc., and Syt1 and DNAJC5 genes were cloned using the pReceiver-LV203 (Genecopoiea Inc., EX-NEG-LV203) vector (Cosmogenetech Inc., using cloning service). The recombinant genes were injected into MAX Efficiency^{™} DH5α Competent Cells (Thermo Fisher, 18258012) and obtained in large quantities using the PureLink^{™} HiPure Plasmid Filter Midiprep Kit (Invitrogen, K210015).

### 3. Cell Culture

Neuro-2a cells were cultured or subcultured using EMEM (Eagle's Minimum Essential Medium) containing 10% complement-inactivated fetal bovine serum (FBS) and 1X antibiotics. In addition, SiMa (DSMZ^{®} ACC 164^{™}) cells were cultured or subcultured using RPMI1640 medium containing 10% complement-inactivated fetal bovine serum and 1X antibiotics. For the experiment, 500 µl/well of PDL coating solution (50 µg/ml poly-D-lysine in 0.1 M borate buffer (pH 8.5)) was added to a 12-well culture dish which was then coated overnight in a biosafety cabinet. Before use, the culture dish was washed twice with 1 ml/well of DPBS (Dulbecco's phosphate-buffered saline). Each cell type was counted and 1 ml of each cell type was seeded at a concentration of 5.5 X 10⁵ cells/ml. The cells were cultured overnight in an incubator at 37°C under 5% CO₂ and then used in the experiment.

### 4. Treatment with Botulinum Toxin (BoNT/A)

A BoNT/A stock solution (Hugel, BA2001, 556 nM) was diluted to 0.5, 5, 25, or 50 pM using an intoxication medium (RPMI 1640, 5 mM arginine (pH 6.0), 2 mM L-alanyl-L-glutamine, 50 µg/mL ganglioside GT1b, 2X N-2 supplement, 2X B-27 supplement, 1X MEM non-essential amino acid, 1X antibiotics). After removing the culture medium from each cell type cultured in the 12-well culture dish, the cells were washed once with 1 ml/well of RPMI 1640 (serum-free) and then treated with 1 ml/well of diluted BoNT/A.

### 5. Transfection and Lentiviral Infection

Target gene transfection was performed immediately after BoNT/A treatment. Cells were transfected with 1 to 2 µg/well of each target gene using Lipofectamine 3000 transfection reagent (Invitrogen, L3000015) and cultured in an incubator at 37°C under 5% CO₂ for 4 days. For lentiviral infection, 1 ml/well of a viral soup diluted at a ratio of 1:3 (viral soup: culture medium, contained 5 µg/mL polybrene) was used. After removing the culture medium from the 12-well culture dish in which the cells were seeded, the cells were infected overnight with the viral soup, and then treated with BoNT/A and cultured in an incubator at 37°C under 5% CO₂ for 3 days. In addition, for the preparation of the lentiviral soup, 293Ta (Gencopoeia, CLv-PK-01) cells as host cells and the Lenti-Pac HIV Expression Packaging Kit (Genecopoeia, LT002) were used.

### 6. Western Blotting

To obtain a total cell lysate (TCL), the cells were washed with 1 ml/well of cold DPBS. The washed cells were lysed with 100 µL/well of lysis buffer (20 mM HEPES (pH 7.5), 1% Triton X-100, 0.2M NaCl, 1 mM EGTA, 5 mM EDTA, protease inhibitor cocktail) and then centrifuged at 13,000 rpm at 4°C for 15 minutes to separate the supernatant. After quantifying the TCL using Bradford assay, a loading sample was prepared at a concentration of 1 mg/ml, and reducing agents and LDS sample buffer were added thereto. The loading sample was separated through SDS-PAGE using a 4-20% gradient SDS-PAGE gel (Bio-Rad, 4561096). Thereafter, the protein was transferred from the SDS-PAGE gel to a PVDF membrane (Bio-Rad, Mini-PROTEAN^{®} Tetra electrophoresis system) which was then washed with washing buffer (TBST, 0.1% Tween-20 in 1X TBS). The membrane was blocked with blocking buffer (5% skim milk in TBST) for 30 minutes and then incubated with each of primary antibodies and secondary antibodies for 45 minutes, and after each step, the membrane was washed three times with washing buffer for 10 minutes per washing. The primary antibodies used in the experiment were anti-SNAP-25 antibody (Sigma, S9684 diluted 1:10000 in blocking buffer), anti-GFP antibody (Abcam, ab290 diluted 1:2000 in blocking buffer), and α-tubulin (DM1A) (Santa Cruz, sc-32293 diluted 1:500 in blocking buffer), and the secondary antibodies were goat anti-rabbit IgG (Abcam, ab97080 diluted 1:10000 or 1:15000 in blocking buffer), anti-mouse IgG, and HRP-linked antibody (Cell Signaling, 7076s diluted 1:1000 in blocking buffer). Finally, to examine the expression of each protein, the membrane was developed using Clarity^{™} western ECL Substrate reagent (Bio-Rad, 1705061) (Bio-Rad, ChemiDoc^{™} MP Imaging system).

### 7. Quantification of Cleavage Percentage of SNAP25 Protein

From the images taken using the ChemiDoc^{™} MP imaging system (Bio-Rad), the band intensity was analyzed using Image lab^{™} software (Bio-Rad), and the Adj. Vol (Int) and percentage of each band were measured.

### Results

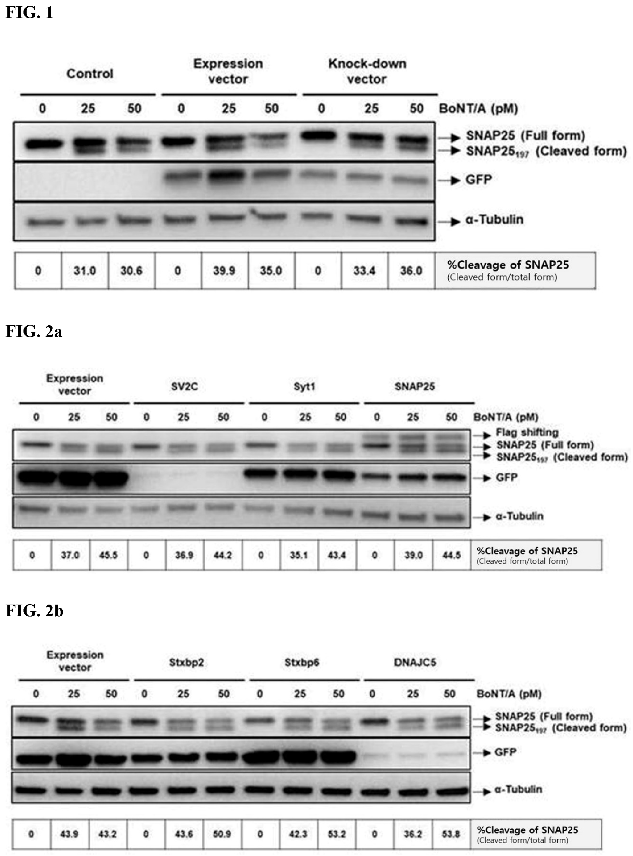

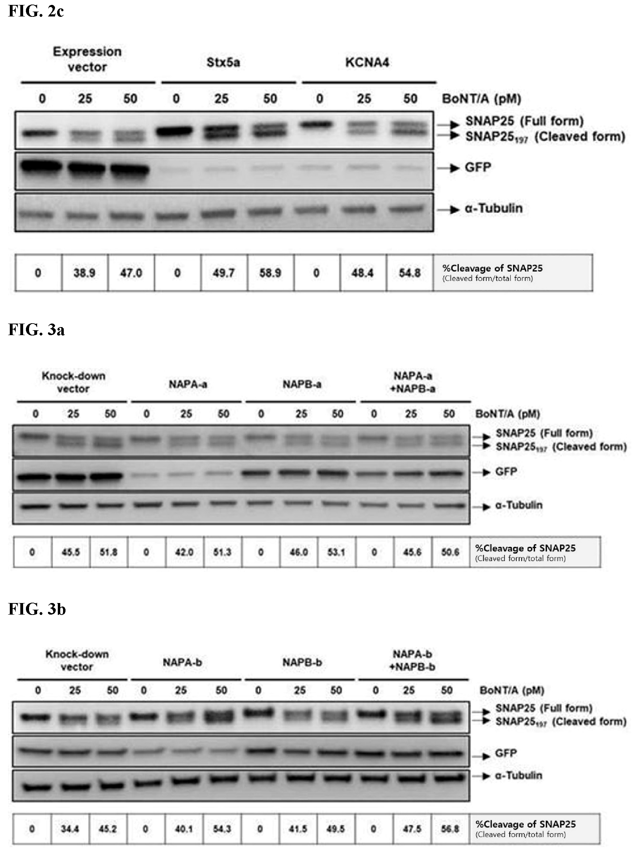

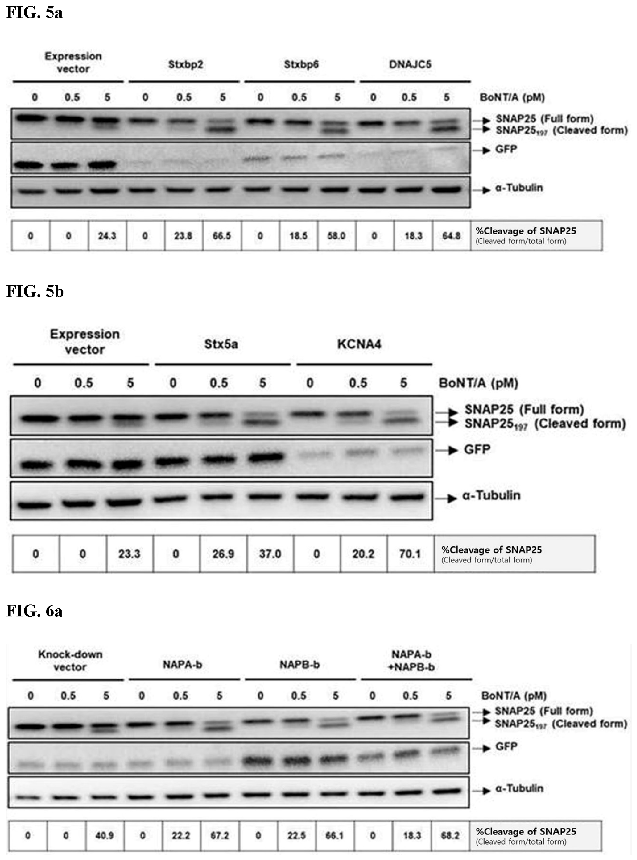

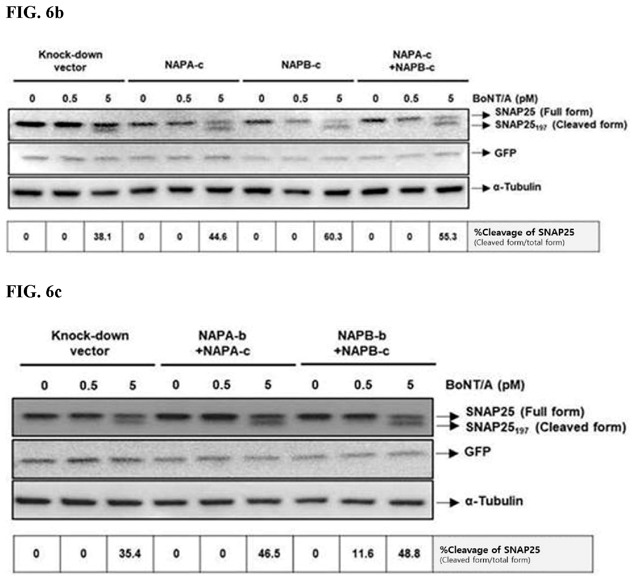

FIGS. 1 to 6 show the results obtained by treating Neuro-2a or SiMa cells, single-transfected or co-transfected with the genes shown in Table 1 above, with botulinum toxin, and determining the percentage of botulinum toxin-induced cleavage of SNAP25 protein in the cells.

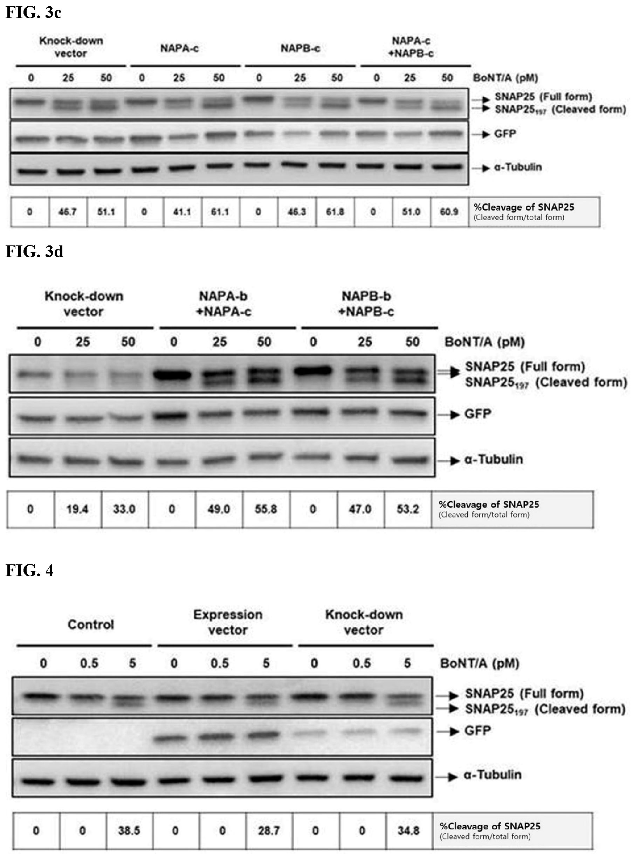

In addition, Tables 2 and 3 below show the results of expressing the value, obtained by quantifying each Western band shown in FIGS. 1 to 6, relative to the value measured for the expression vector or knock-down vector of the control group (FIG. 1 or 4).

**[Table 2]**

| Cells: Neuro-2a | | | | | | Percent increase in sensitivity compared to control (50 pM BoNT/A) |
|---|---|---|---|---|---|---|
| BoNT/A treatment concentration (pM) | | | 0 | 25 | 50 | |
| [FIG. 2A] | Expression vector (Control) | | 0 | 37.0 | 45.5 | - |
| | UP | SV2C | 0 | 36.9 | 44.2 | 97.14 % |
| | UP | Syt1 | 0 | 35.1 | 43.4 | 95.38 % |
| | UP | SNAP25 | 0 | 39.0 | 44.5 | 97.80 % |
| [FIG. 2B] | Expression vector (Control) | | 0 | 43.9 | 43.2 | - |
| | UP | Stxbp2 | 0 | 43.6 | 50.9 | 117.82 % |
| | UP | Stxbp6 | 0 | 42.3 | 53.2 | 123.15 % |
| | UP | DNAJC5 | 0 | 36.2 | 53.8 | 124.54 % |
| [FIG. 2C] | Expression vector (Control) | | 0 | 38.9 | 47.30 | - |
| | UP | Stx5a | 0 | 49.7 | 58.9 | 124.52 % |
| | UP | KCNA4 | 0 | 48.4 | 54.8 | 115.86 % |
| [FIG. 3A] | Knock-down vector (Control) | | 0 | 45.5 | 51.8 | - |
| | DOWN | NAPA-a | 0 | 42.0 | 51.3 | 99.03 % |
| | DOWN | NAPB-a | 0 | 46.0 | 53.1 | 102.51 % |
| | DOWN | NAPA-a + NAPB-a | 0 | 45.6 | 50.6 | 97.68 % |
| [FIG. 3B] | Knock-down vector (Control) | | 0 | 34.4 | 45.2 | - |
| | DOWN | NAPA-b | 0 | 40.1 | 54.3 | 120.13 % |
| | DOWN | NAPB-b | 0 | 41.5 | 49.5 | 109.51 % |
| | DOWN | NAPA-b + NAPB-b | 0 | 47.5 | 56.8 | 125.66 % |
| [FIG. 3C] | Knock-down vector (Control) | | 0 | 46.7 | 51.1 | - |
| | DOWN | NAPA-c | 0 | 41.1 | 61.1 | 119.57 % |
| | DOWN | NAPB-c | 0 | 46.3 | 61.8 | 120.94 % |
| | DOWN | NAPA-c + NAPB-c | 0 | 51.0 | 60.9 | 119.18 % |
| [FIG. 3D] | Knock-down vector (Control) | | 0 | 19.4 | 33.0 | - |
| | DOWN | NAPA-b + NAPA-c | 0 | 49.0 | 55.8 | 169.09 % |
| | DOWN | NAPB-b + NAPB-c | 0 | 47.0 | 53.2 | 161.21 % |

**[Table 3]**

| Cells: SiMa | | | | | | Percent increase in sensitivity compared to control (5 pM BoNT/A) |
|---|---|---|---|---|---|---|
| BoNT/A treatment concentration (pM) | | | 0 | 0.5 | 5 | |
| [FIG. 5A] | Expression vector (Control) | | 0 | 0 | 24.3 | - |
| | UP | Stxbp2 | 0 | 23.8 | 66.5 | 273.66 % |
| | UP | Stxbp6 | 0 | 18.5 | 58 | 238.68 % |
| | UP | DNAJC5 | 0 | 18.3 | 64.8 | 266.67 % |
| [FIG. 5B] | Expression vector (Control) | | 0 | 0 | 23.3 | - |
| | UP | Stx5a | 0 | 26.9 | 37 | 158.80 % |
| | UP | KCNA4 | 0 | 20.2 | 70.1 | 300.86 % |
| [FIG. 6A] | Knock-down vector (Control) | | 0 | 0 | 40.9 | - |
| | DOWN | NAPA-b | 0 | 22.2 | 67.2 | 164.30 % |
| | DOWN | NAPB-b | 0 | 22.5 | 66.1 | 161.61 % |
| | DOWN | NAPA-b + NAPB-b | 0 | 18.3 | 68.2 | 166.75 % |
| [FIG. 6B] | Knock-down vector (Control) | | 0 | 0 | 38.1 | - |
| | DOWN | NAPA-c | 0 | 0 | 44.6 | 117.06 % |
| | DOWN | NAPB-c | 0 | 0 | 60.3 | 158.27 % |
| | DOWN | NAPA-c + NAPB-c | 0 | 0 | 55.3 | 145.14 % |
| [FIG. 6C] | Knock-down vector (Control) | | 0 | 0 | 35.4 | - |
| | DOWN | NAPA-b + NAPA-c | 0 | 0 | 46.5 | 131.36 % |
| | DOWN | NAPB-b + NAPB-c | 0 | 11.6 | 48.8 | 137.85 |

In Tables 2 and 3 above, if the percent increase in sensitivity is >100%, it can be determined that the sensitivity increased. The experimental results indicated that when the Neuro-2a cells were transformed so that the Stxbp2, Stxbp6, DNAJC5, Stx5a, or KCNA4 gene would be overexpressed, or when the Neuro-2a cells were transformed so that NAPB-a, NAPA-b, NAPB-b, NAPA-b + NAPB-b, NAPA-c, NAPB-c, NAPA-c + NAPB-c, NAPA-b + NAPA-c, or NAPA-b + NAPA-c genes would be silenced, the sensitivity of the cells to botulinum toxin increased (based on 50 pM BoNT/A). In addition, it was shown that when the SiMa cells were transformed so that the Stxbp2, Stxbp6, DNAJC5, Stx5a, or KCNA4 gene would be overexpressed, or when the SiMa cells were transformed so that NAPA-b, NAPB-b, NAPA-b + NAPB-b, NAPA-c, NAPB-c, NAPA-c + NAPB-c, NAPA-b + NAPA-c, or NAPB-b + NAPB-c genes would be silenced, the sensitivity of the cells to botulinum toxin increased (based on 5 pM BoNT/A).

From the above results, it could be seen that when the Stxbp2, Stxbp6, DNAJC5, Stx5a, or KCNA4 gene selected in the present invention was overexpressed or when NAPA and NAPB were silenced alone or in combination, the sensitivity of the cells to botulinum toxin significantly increased.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

## Claims

1. A neuronal cell line sensitive to botulinum toxin, wherein Stxbp6 is overexpressed compared to that gene in a parent cell line, wherein the parent cell line is a Neuro-2a cell line or a SiMa cell line.

2. The cell line according to claim 1, wherein the botulinum toxin is botulinum toxin serotype A.

3. The cell line according to claim 1, which is for determination of activity of the botulinum toxin or detection of the botulinum toxin.

4. A method for producing a neuronal cell line sensitive to botulinum toxin, the method comprising overexpressing Stxbp6 in an isolated neuronal cell line, wherein the isolated neuronal cell line, as the parent cell line, is a Neuro-2a cell line or a SiMa cell line.

5. The method according to claim 4, wherein the botulinum toxin is botulinum toxin serotype A.

6. A cell-based method for determining activity of botulinum toxin, the method comprising:
culturing the cell line according to any one of claims 1 to 3;
treating the cultured cell line with the botulinum toxin; and
measuring sensitivity of the botulinum toxin-treated cell line to the botulinum toxin.

7. The cell-based method according to claim 6, wherein the botulinum toxin is botulinum toxin serotype A.

8. The cell-based method according to claim 6, wherein the measuring the sensitivity of the botulinum toxin-treated cell line to the botulinum toxin comprises measuring cleavage of endogenous neurosecretory protein.

9. A cell-based method for detecting botulinum toxin, the method comprising:
culturing the cell line according to any one of claims 1 to 3;
treating the cultured cell line with a sample of interest; and
measuring sensitivity of the sample-treated cell line to the botulinum toxin.

10. The cell-based method according to claim 9, wherein the botulinum toxin is botulinum toxin serotype A.

11. The cell-based method according to claim 9, wherein the measuring the sensitivity of the sample-treated cell line to the botulinum toxin comprises measuring cleavage of endogenous neurosecretory protein.
